# EUROPEAN PATENT SPECIFICATION

(11) **EP 1 545 653 B1**
(45) Date of publication and mention of the grant of the patent: **13.09.2006**
(21) Application number: 03766943.9
(22) Date of filing: 29.07.2003
(51) Int. Cl.: A61M 3/02, A61M 1/00, A61F 5/44

(54) **NOZZLE FOR STOMA CLEANSING SYSTEM**
DÜSE FÜR EIN STOMA-REINIGUNGSSYSTEM
BUSE POUR SYSTEME DE NETTOYAGE DE STOMIE

(30) Priority: 02.08.2002 US 211949
(43) Date of publication of application: 29.06.2005
(73) Proprietor: Zassi Medical Evolutions, Inc., Fernandina Beach, FL 32034 (US)
(72) Inventor: LEBER, Leland, C., Fort Collins, CO 80526 (US); VON DYCK, Peter, M., Fernandina Beach, FL 32034 (US); SCHNEIDER, James, G., Chesterfield, MO 63017 (US); WHITE, Steven, Wellington, FL 33414 (US)
(74) Representative: Thacker, Darran Ainsley
(86) International application number: PCT/US2003/023609
(87) International publication number: WO 2004/012792

(56) References cited:
- EP-A- 0 456 470
- WO-A-96/29044
- DE-A- 3 430 095
- US-A- 1 845 343
- US-A- 2 243 299
- US-A- 4 294 251
- US-A- 4 682 979
- US-A- 4 804 373
- US-A- 5 441 482

## Description

### FIELD OF THE INVENTION

The present invention relates generally to a system for cleansing an internal body cavity, and, more particularly, to a system for an irrigating a colon through a to remove fecal material.

### BACKGROUND OF INVENTION

It is well known that individuals having surgically created stomas have some special needs associated with evacuation of bowel contents through the stoma. The need for a gentle irrigation method that aggressively breaks up intestinal contents without damaging the fragile intestinal lining has prevented prior devices from being able to successfully and safely facilitate rapid bowl evacuation. Irrigation, especially on a repeated basis, can compromise mucous linings, resulting in actinic changes to mucous membrane tissue. Use of known systems for irrigation of a body cavity, typically a colon, takes a long time, which can be very disruptive of the user's life-style. It is therefore desirable to provide a system that produces gentle irrigation, while at the same time permitting aggressive break up of the matter to be removed.

Attempts to develop systems that are considered to be gentle on tissue typically consist of gravity feed bags with a single lumen and steady flow rate nozzle. These systems are very slow and generally used by caregivers, rather than by the patient for irrigation.

A known electro-mechanical system, Pulsed Irrigation and Evacuation (PIE), pumps a very large volume of water (such as about 11.5 - to about 19 liters) at a flow rate of two to three liters per minute. The water is pumped into the colon through a specula inserted into the rectum or stoma. The water is pumped in in aliquots (25 - 1―ml) that repeatedly fill and empty the colon at a very rapid rate (e.g. one to four second cycles). This system is far too aggressive to be used on a regular basis, except in sever cases of chronic bowel impaction, where alternatives are limited. These cases include patients with chronic neurogenic bowel due to spinal chord injury or disease. Thus, the PIE system is too aggressive for widespread use, and is limited to cases where other, less aggressive or less invasive alternatives do not exist. The present invention provides a mechanism for addressing the above problems.

U.S. Patent 4,294,251 discloses a suction lavage system that can be used to improve the interface between a prosthesis, such as a hip replacement, and the interdigitating network of cancellous bone in which it is secured. The system comprises a probe having a water conduit generally located outside a suction conduit. The outer surface of probe is provided with apertures to allow the water in the water conduit to exit the probe in the form of pulsatile jets. The openings can be arranged such that the jets are at right angles to the longitudinal axis of the water conduit or parallel to it.

### SUMMARY OF INVENTION

An aspect of the present invention, briefly, is a cleansing system for the intestine of a patient.

An aspect of the present invention is to provide a nozzle assembly as defined in claim 1. The nozzle assembly is included in the apparatus of claim 9.

These and other aspects and advantages of the invention will be in part apparent and in part pointed out herein below. These are merely illustrative aspects of the present invention and should not be deemed an all-inclusive listing of the innumerable aspects associated with the present invention.

### BRIEF DESCRIPTION OF DRAWINGS

FIG. 1 is a perspective view of the nozzle assembly of the present invention.
FIG. 2 is a cross-sectional view taken along line 2-2 of FIG. 1.
FIG. 3 is a perspective view of the interior of transition hub of the nozzle assembly of FIG. 1.
FIG. 4 is a perspective view of the exterior of the transition hub of FIG. 3.
FIG. 5 is a perspective view of the interior of the hubcap of the nozzle assembly of FIG. 1.
FIG. 6 is a perspective view of the exterior of the hubcap of FIG. 5.
FIG. 7 is a perspective view of the distal end of the nozzle assembly of FIG. 1 illustrating an embodiment having three lumens.
FIG. 8 is a perspective view of the distal end of the nozzle assembly of FIG. 1 illustrating an embodiment having two lumens.
FIG. 9 is a perspective view of the distal end of the nozzle assembly of FIG. 1 illustrating an embodiment having four lumens.
FIG. 10 is a cross-sectional view illustrating the angular projection of the lumen in a preferred embodiment.
FIG. 11 is a perspective view of the irrigation tubing assembly of the present invention.
FIG. 12 is an exploded view of the irrigation tubing assembly of FIG. 11.
FIG. 13 is a cross-sectional view along line 13-13 of FIG. 11.

### DETAILED DESCRIPTION

The present invention is a cleansing system apparatus for irrigation of a cavity for the removal of solid and colloidal matter. This invention has utility to a wide variety of industrial, medical and cosmetic applications. Without straying from the broad intent of this invention, a medical application of the technology will be used for illustration.

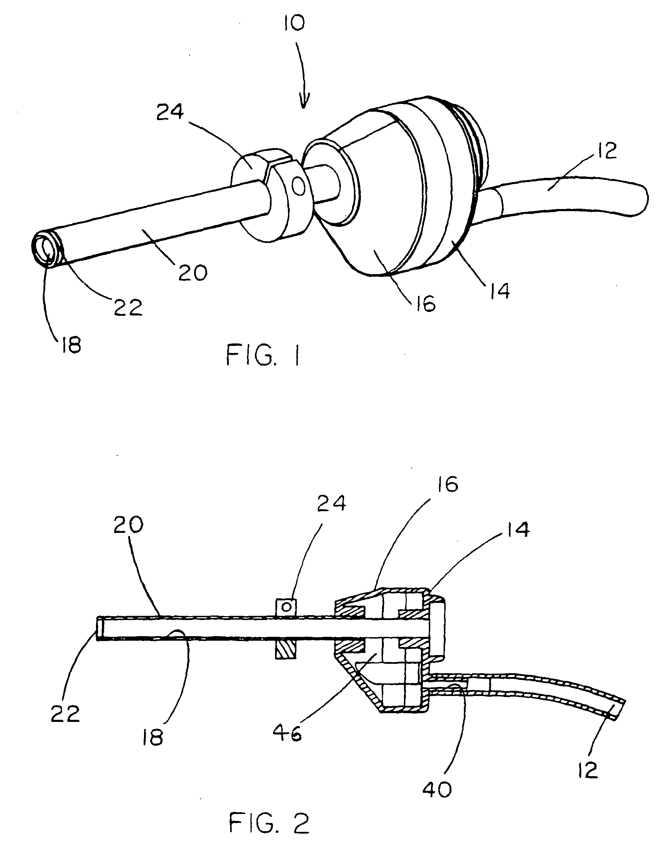

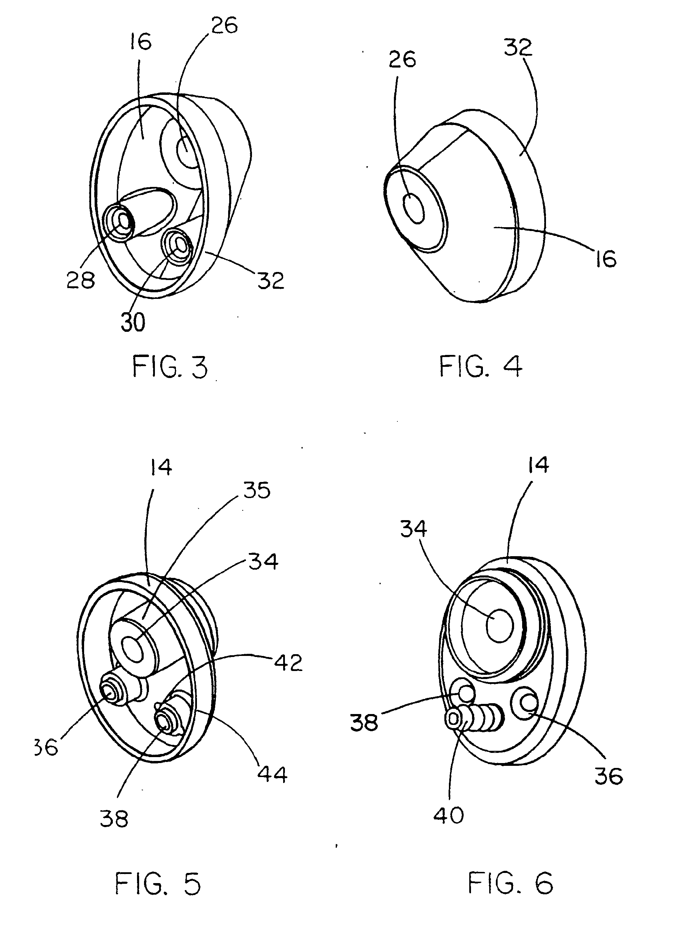

A nozzle assembly, generally designated 10, is shown in FIGS. 1 and 2. Nozzle assembly 10 comprises the main component of a delivery system. Nozzle assembly 10 is connected to a pumping system, not shown, by a polyethylene tube, 12. Assembly 10 further includes a transition hubcap 14 and a transition hub 16, shown in more detail in FIGS. 3 through 6, discussed below. Inner and outer nozzle tubes 18, 20 respectively, nozzle tip 22 and location collar 24 complete the basic nozzle assembly 10.

FIGS. 3 and 4 illustrate the transition hub 16 in detail. Transition hub 16 includes a central aperture 26, threaded screw acceptors 28 and 30 and a shoulder 32. FIGS. 5 and 6 illustrate transition hubcap 14, including a central aperture 34 and associated projecting shoulder 35, and screw apertures 36 and 38. Transition hubcap 14 further includes a cleansing fluid inlet port 42, an opposing projection 40, and a shoulder 44.

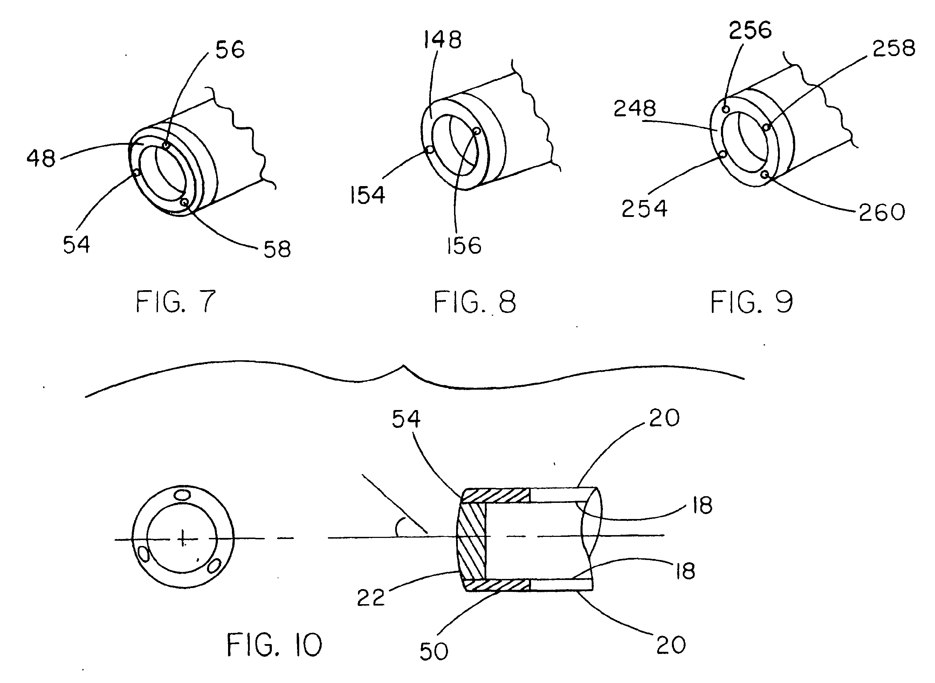

Nozzle tip 22 includes a front face 48, an inner projecting portion 52 and an outer projecting portion 50, best seen in FIGS. 7 through 10. A variety of nozzle assemblies have been devised that demonstrate varying levels of performance and can be selected from for use, depending upon the particular application. In a first embodiment, illustrated in FIG. 7, the front face 48 includes three apertures, or lumens 54, 56 and 58, formed as a result of cutting or machining fluid lumens in the nozzle body. The angle of the lumens relative the axis of the nozzle tubes 18, 20, is shown in FIG. 10. This angle results in discharge of the fluid stream from nozzle tip 22 at a preferred 60° angle relative to the axis of the nozzle tubes 18, 20. An acceptable range for the angle of the discharge of fluid stream relative to the axis of the nozzle tubes is within about 30° to about 60°. The purpose of this angle is to impart a swirling action to the fluid as it exits the nozzle assembly, and may be varied depending on the particular application. The fluid discharging lumens are fed pulses intermittently in sequence or simultaneously, as preferred. This provides a swirling ("vortex") action that imparts a shearing action to the matter to be broken up, which causes it to break into clumps. Elements which are particularly critical in creating this vortex effect are: 1) the angulation of the orifices, combined with 2) pulsatile (sequential) flow and 3) a plurality of angled lumens and 4) the energy of the fluid streams. The intermittent action of the pulse provides a "jack hammer" action to further break up the clumps. Similarly, with only simultaneous flow out of the same nozzle design there is created only the "jack hammer" action, which has not proven to be as effective in breaking up clumps if all other parameters (i.e. orifice angle, number of orifices and energy of fluid streams) remain constant.

Another factor which importantly affects the effectiveness of the fluid stream in breaking up fecal clumps is the size of the lumen orifices. In order for the proper nozzle stream velocity to be obtained the orifices should be in the range of about 1.0 mm to about 0.5 mm in diameter. The overall construction of the transition hub and hubcap (the "manifold" system) is designed to provide equivalent flow out of each orifice. The integration of the nozzle with the manifold is constructed to provide such equivalent flow.

In an alternative embodiment, this nozzle assembly may be fitted with an additional lumen to provide access to the body cavity into which the nozzle is placed for the purpose of facilitating the monitoring pressure or temperature. Particularly the monitoring of pressure within the body cavity as necessary for safety reasons, to prevent the possible rupture of the body cavity.

In another embodiment, illustrated in FIG. 8, front face 148 includes two apertures, or lumens, 154, 156, located opposite each other on the periphery of the nozzle tube. These are fed fluid by the pumping system in either alternating intermittent or simultaneous pulses. Use of this nozzle permits matter to be broken up by "batting" it back and forth with the pulses of fluid. In an alternate embodiment, not shown, this nozzle assembly may also include a lumen to provide access to the body cavity into which the nozzle is placed for the purpose of facilitating the monitoring pressure or temperature, as described above.

In yet another embodiment, illustrated in FIG. 9, front face 248 includes four apertures or lumens 254, 256, 258 and 260 equally spaced apart around the periphery of the nozzle exit. This nozzle configuration permits aggressive pulsating application of fluid to permit effective break up of the contents of a body cavity without damage to the neighboring tissue. In an alternative embodiment, not shown, the nozzles are preferably arranged in two pairs, each pair being operated in sequence with the opposite pair. The first pair imparts a clockwise swirl to the fluid, while the second pair imparts a counterclockwise flow to the fluid. The resultant action of operating these pairs of pulsejets in and out of phase sequence is to impart additional shear to the target matter resulting in rapid breakup of the target material. This assembly can also be fitted with pressure/temperature sensing/communicating lumens and a central drain tube as in the other nozzle assemblies. Other constructions and variation of the described nozzles and pumping systems can be conceived which are considered to be within the scope of the invention, such as varying the number of lumens in each set.

As is best seen in the cross-sectional view FIG. 2, the irrigation tube 12 is connected to transition hubcap 14 through projection 40. Transition hub 16 and hubcap 14 are attached to each other along shoulders 32 and 44, respectively, by a silicon seal, not shown, and are held in place by screws extending through screw apertures 36, 38 into screw acceptors 28, 30. Transition hub 16 and hubcap 14 form a nozzle body that houses a cavity 46, the cavity being is in fluid communication with irrigation tubing 12 through projection 40.

Central apertures 26, 34 accept the inner nozzle tube 18, which is open to the atmosphere through central aperture 34. The interior of inner nozzle tube 18 and central aperture 34 may be adapted to provide a relatively large central drain hole in the middle of the nozzle assembly that can be gravity fed, pressure fed or vacuum evacuated, as desired. The purpose of such adaptation is to drain the matter broken up by the nozzle along with the irrigation fluid from the body cavity. Outer nozzle tube is located coaxially around inner nozzle tube, and is sealed at the projecting shoulder 35 of hubcap 14. The coaxial arrangement of inner and outer nozzle tubes 18, 20 forms an annular space, that is in fluid communication with cavity 46. Nozzle tubes 18, 20 are connected to nozzle tip projecting portion 50, seen in FIG. 10, by any suitable means, for example by silver solder bonding when the components are formed from metal.

In operation, a cleansing fluid stream is provided to the nozzle assembly through irrigation tubing 12 and into cavity 46 through projection 40. The fluid fills cavity 46 and is then forced into annular flow channel 48 and discharged through nozzle tip lumens 54, 56, 58. This nozzle system also provides for the draining of the irrigated area through a relatively large drain tube that can be inserted into inner tube 18 through central aperture 34. The drain tube can be left open or intermittently closed as conditions warrant.

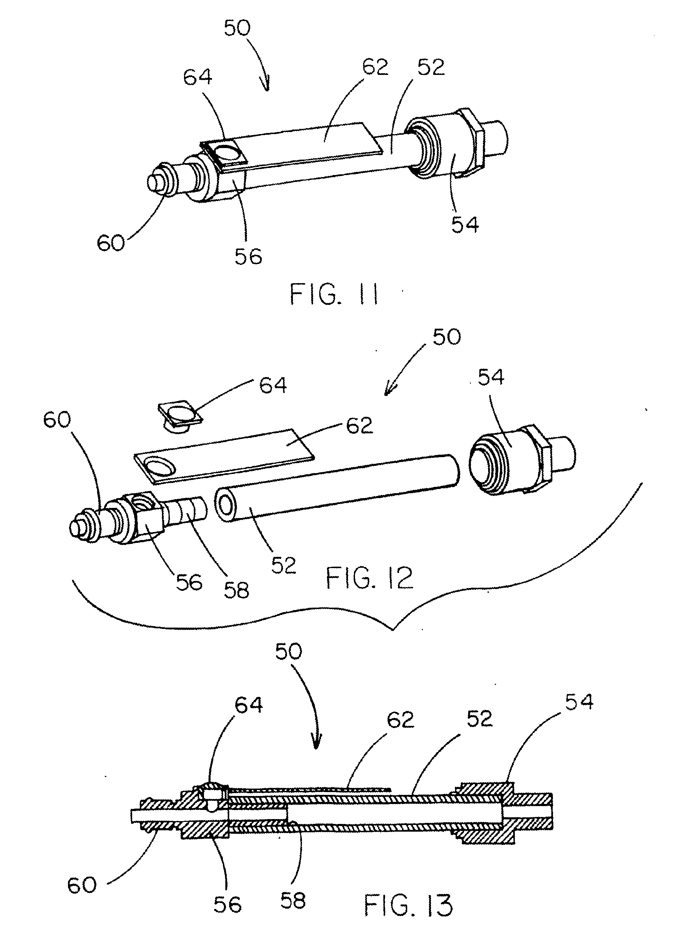

Attached downstream of irrigation tubing 12 is an irrigation tubing assembly generally designated 50, shown in FIGS. 11 through 13. Tubing assembly 50 allows the internal pressure to be monitored in order to detect flow blockage or leakage.

Tubing 52 is attached to connector 54, pressure sensor housing 56, connectors 58, 60, circuit board 62 and pressure sensor 64. The electronic pressure monitor and associated circuit board may be of any conventional design, as is well known by those in the art.

Nozzle assembly 10 is connected to a pumping system, not shown, through irrigation tubing assembly 50. In one embodiment, the new system utilizes a peristaltic pumping mechanism, which insures hygienic fluid delivery. This system may provide a motor controller capable of on/off temperature control of the pump mechanism and an internal pressure monitoring system for pump flow blockage or leakage detection. This monitoring system may be coupled with an intra-lumenal pressure sensor referenced to atmospheric pressure for the purpose of monitoring the anatomical cavity pressures associated with the nozzle portion of the device. An example of this type of pumping system is described in U.S. Patent Ser. No. 09/362,638, filed July 28, 1999.

Suitable pumping systems provide for independent adjustment of pressure and flow rate as well as adjustment of pulse rate. Other adjustments may include flow rate, pulse duration, pulse volume, total volume, time between pulses or dwell time. Preset parameters may include pressure of fluid delivery, fluid velocity as dictated by number, size and orifice geometry of flow lumens at any given flow rate, and temperature of liquid being delivered.

An alternate embodiment of the pumping system generates fluid pressure, which is stored in a bladder type tank. In this case the pump is controlled by an adjustable pressure-sensing switch. The fluid is directed from the bladder storage tank to a motor operated pulsing mechanism. One embodiment includes a series of cam operated valves, the inputs of which are connected by a fluid bus to the bladder tank. The output from each valve is independently directed to the fluid delivery nozzle assembly through separate tubes. The duty cycle of each pulse is controlled by a combination of the cam design and the location of the valves relative to the cam. One embodiment has two separate cams and sets of valves, provided to allow the use of any of the nozzle assemblies. The cams of this embodiment are motor-operated with a motor the speed of which is user controllable. This embodiment also includes a tachometer to measure and display the rotational speed of the cam, thus allowing the user to precisely adjust the speed of the pulses. An additional feature is the ability to provide a steady flow to all of the fluid conduits simultaneously. Additionally, this embodiment also provides the ability to have a user-selectable off-dwell period during which no pumping occurs.

While specific embodiments have been shown and described, many variations are possible. Additional variations within the scope of the invention are as follows. Nozzle bodies can be formed of rigid fabrication or flexible material fabrication, or combinations thereof to achieve conformance to passages (such as a stoma tract) used for nozzle insertion, drain configurations and fluid lumen geometry. Fluid lumens may be of separate tubing configurations, further defined as a nozzle by jacketing, joining or insert molding such tubing sections into nozzle forms.

Additionally, the nozzle body can be integral to a catheter, stint or port structure (see, e.g., U.S. Patent 6,033,390) wherein the catheter, stint or port provides a permanent or semi-permanent fluid communication between the body cavity and the atmosphere and is connected to the pumping device via the irrigation set when it is desired to irrigate the body cavity.

Nozzle orifices have been defined as being formed as a result of cutting or machining fluid lumens in a nozzle body material and exposing lumen openings for fluid escapement. However, lumens may also be formed from separate components inserted into nozzle lumens to effect a defined orifice and pattern of fluid escapement. Such orifice articles are easily built in plastics and metals.

Pump sequences can include the continuous or intermittent delivery of fluids with uninterrupted drain flow, periodic drain flow, or no drain flow until delivery of fluids is complete. Dwell periods may be utilized for further dissolution and wetting of effected masses at any stage of the pump sequence wherein pulsation or supply flow is not occurring.

While suitable materials for constructing the present invention have been disclosed, the components may be made of any suitable, medically acceptable material.

## Claims

1. A nozzle assembly (10) comprising:
a nozzle body (14,16) housing a nozzle body cavity (46);
an inlet port (40) in fluid communication with the nozzle body cavity (46);
an annular fluid channel in fluid communication with the nozzle body cavity (46); and
a nozzle tip (22) having at least two lumens (154,156; 54,56,58; 254,256,258,260) in fluid communication with the fluid channel and arranged at an angle relative to the annular fluid channel **CHARACTERISED IN THAT** the angle of the at least two lumens relative to the annual fluid channel is between 30° and 60° so as to discharge a swirling stream of irrigation fluid into the body cavity of a patient into which the nozzle assembly (10) is inserted in use.

2. A nozzle assembly (10) according to claim 1, wherein the nozzle tip (22) includes two lumens (154,156).

3. A nozzle assembly (10) according to claim 1, wherein the nozzle tip includes three lumens (54,56,58).

4. A nozzle assembly (10) according to claim 1, wherein the nozzle tip includes four lumens (254,256,258,260).

5. A nozzle assembly (10) according to any preceding claim, wherein the at least two lumens have a diameter of between 0.5 mm and 1.0 mm.

6. A nozzle assembly (10) according to any preceding claim, wherein the nozzle body and the nozzle body cavity (46) are formed by the interconnection of a transition hub (16) and a transition hubcap (14).

7. A nozzle assembly (10) according to any preceding claim, wherein the annular fluid channel is formed between an inner nozzle tube (18) and an outer nozzle tube (20) in coaxial arrangement.

8. A nozzle assembly (10) according to any preceding claim, further comprising an additional lumen within the nozzle assembly (10) to act as a conduit through which the internal pressure or temperature of the body cavity of the patient can be monitored in use.

9. An apparatus for cleaning the body cavity of a patient comprising:
a nozzle assembly (10) according to any preceding claim; and
a pump adapted to provide pulses of irrigation fluid at a controlled rate to the inlet port (40) of the nozzle assembly (10).

10. An apparatus according to claim 9, wherein the pump is a peristaltic pump.

## Patentansprüche

1. Düsenanordnung (10, mit:
einem Düsenkörper (14, 16), der einen Düsenkörperhohlraum (46) beherbergt,
einem Einlasskanal (40) in Fluidverbindung mit dem Düsenkörperhohlraum (46),
einem ringförmigen Fluidkanal in Fluidverbindung mit dem Düsenkörperhohlraum (46), und
einer Düsenspitze (22), die wenigstens zwei Lumen (154, 156; 54, 56, 58; 254, 256, 258, 260) in Fluidverbindung mit dem Fluidkanal und unter einem Winkel bezüglich des ringförmigen Fluidkanals angeordnet aufweist,
**dadurch gekennzeichnet, dass** der Winkel der wenigstens zwei Lumen bezüglich des ringförmigen Fluidkanals zwischen 30° und 60° beträgt, um einen wirbelnden Strom von Spülfluid in die Körperhöhlung eines Patienten abzugeben, in die die Düsenanordnung (10) bei Benutzung eingeführt ist.

2. Düsenanordnung (10) nach Anspruch 1, bei der die Düsenspitze (22) zwei Lumen (154, 156) umfasst.

3. Düsenanordnung (10) nach Anspruch 1, bei der die Düsenspitze drei Lumen (54, 56, 58) umfasst.

4. Düsenanordnung (10) nach Anspruch 1, bei der die Düsenspitze vier Lumen (254, 256, 258, 260) umfasst.

5. Düsenanordnung (10) nach einem der vorhergehenden Patentansprüche, bei der die wenigstens zwei Lumen einen Durchmesser von zwischen 0,5 mm und 1,0 mm haben.

6. Düsenanordnung (10) nach einem der vorhergehenden Ansprüche, bei der der Düsenkörper und der Düsenkörperhohlraum (46) durch die Verbindung einer Übergangsnabe (16) mit einem Übergangsnabendeckel (14) gebildet werden.

7. Düsenanordnung (10) nach einem der vorhergehenden Ansprüche, bei der der ringförmige Fluidkanal zwischen einem inneren Düsenrohr (18) und einem äußeren Düsenrohr (20) in koaxialer Anordnung gebildet ist.

8. Düsenanordnung (10) nach einem der vorhergehenden Ansprüche, ferner umfassend ein zusätzliches Lumen innerhalb der Düsenanordnung (10), welches als eine Leitung agiert, durch die der innere Druck oder die Temperatur der Körperhöhlung des Patienten im Gebrauch überwacht werden kann.

9. Vorrichtung zum Reinigen der Körperhöhlung eines Patienten, mit:
einer Düsenanordnung (10) nach einem der vorhergehenden Ansprüche, und
einer Pumpe, die Spülfluidpulse mit einer gesteuerten Rate zu dem Einlasskanal (40) der Düsenanordnung (10) zu liefern vermag.

10. Vorrichtung nach Anspruch 9, bei der die Pumpe eine peristaltische Pumpe ist.

## Revendications

1. Ensemble de buse (10), comprenant :
un corps de buse (14, 16) renfermant une cavité de corps de buse (46);
un orifice d'entrée (40) en communication de fluide avec la cavité de corps de buse (46) ;
un canal de fluide annulaire en communication de fluide avec la cavité de corps de buse(46) ; et
une pointe de buse (22) comportant au moins deux conduits (154, 156 ; 54, 56, 58 ; 254, 256, 258, 260) en communication de fluide avec le canal de fluide et agencés selon un certain angle par rapport au canal de fluide annulaire, **caractérisé en ce que** l'angle des conduits au nombre d'au moins deux par rapport au canal de fluide annulaire est compris entre 30° et 60° de façon à délivrer un courant tourbillonnant de fluide d'irrigation dans la cavité corporelle d'un patient dans lequel l'ensemble de buse (10) est inséré lors de l'utilisation.

2. Ensemble de buse (10) selon la revendication 1, dans lequel la pointe de buse (22) comprend deux conduits (154, 156).

3. Ensemble de buse (10) selon la revendication 1, dans lequel la pointe de buse comprend trois conduits (54, 56, 58).

4. Ensemble de buse (10) selon la revendication 1, dans lequel la pointe de buse comprend quatre conduits (254, 256, 258, 260).

5. Ensemble de buse (10) selon l'une quelconque des revendications précédentes, dans lequel les conduits au nombre d'au moins deux ont un diamètre compris entre 0,5 mm et 1,0 mm.

6. Ensemble de buse (10) selon l'une quelconque des revendications précédentes, dans lequel le corps de buse et la cavité de corps de buse (46) sont formés par l'interconnexion d'un moyeu de transition (16) et d'un capuchon de moyeu de transition (14).

7. Ensemble de buse (10) selon l'une quelconque des revendications précédentes, dans lequel le canal de fluide annulaire est formé entre un tube de buse intérieur (18) et un tube de buse extérieur (20) en agencement coaxial.

8. Ensemble de buse (10) selon l'une quelconque des revendications précédentes, comprenant de plus un conduit additionnel à l'intérieur de l'ensemble de buse (10) pour agir en tant que conduit à travers lequel la pression ou la température internes de la cavité corporelle du patient peuvent être contrôlées lors de l'utilisation.

9. Dispositif pour nettoyer la cavité corporelle d'un patient, comprenant :
un ensemble de buse (10) selon l'une quelconque des revendications précédentes ; et
une pompe adaptée pour délivrer des impulsions de fluide d'irrigation à un débit contrôlé à l'orifice d'entrée de l'ensemble de buse (10).

10. Dispositif selon la revendication 9, dans lequel la pompe est une pompe péristaltique.
